# NEUE EUROPÄISCHE PATENTSCHRIFT

(11) **EP 1 322 156 B3**
(45) Veröffentlichungstag dieser Patentschrift: **01.12.2010**
(45) Hinweis auf die Patenterteilung: 15.03.2006
(21) Anmeldenummer: 01985228.4
(22) Anmeldetag: 18.09.2001
(51) Int. Cl.: A01N 33/08, A01N 39/00, A01N 33/12, A01N 33/04

(54) **DESINFEKTIONSMITTEL**
DISINFECTANT
DÉSINFECTANT

(30) Priorität: 20.09.2000 EP 00120590
(43) Veröffentlichungstag der Anmeldung: 02.07.2003
(60) Teilanmeldung: 06004673.7
(73) Patentinhaber: Lonza AG, 4052 Basel (CH)
(72) Erfinder: LICHTENBERG, Florian, 79639 Grenzach-Wyhlen (DE); LüTZELER, Michael, 79639 Grenzach-Wyhlen (DE); RANFT, Volker, 79730 Murg (DE)
(86) Internationale Anmeldenummer: PCT/EP2001/010754
(87) Internationale Veröffentlichungsnummer: WO 2002/023990

(56) Entgegenhaltungen:
- EP-A- 0 333 143
- EP-A- 1 025 967
- WO-A-93/15173
- WO-A-98/20732
- WO-A-99/15012
- FR-A- 2 602 955
- DATABASE WPI Section Ch, Week 199946 Derwent Publications Ltd., London, GB; Class A97, AN 1999-541231 XP002190404 & CN 1 222 566 A (CHEN Y), 14. Juli 1999 (1999-07-14)
- DATABASE WPI Section Ch, Week 199824 Derwent Publications Ltd., London, GB; Class D22, AN 1998-266961 XP002190405 & JP 10 087410 A (KANTO KAGAKU KK), 7. April 1998 (1998-04-07)
- DATABASE WPI Section Ch, Week 197749 Derwent Publications Ltd., London, GB; Class D22, AN 1977-87079Y XP002190406 & JP 50 132126 A (ANONYMOUS), 20. Oktober 1975 (1975-10-20)
- DATABASE CA [Online] CHEMICAL ABSTRACTS SERVICE, COLUMBUS, OHIO, US; retrieved from STN-INTERNATIONAL, accession no. 105:99132 CA XP002190402 & RO 87 920 A (INTREPRINEREA DE DETERGENTI) 20. Dezember 1985 (1985-12-20)
- DATABASE CA [Online] CHEMICAL ABSTRACTS SERVICE, COLUMBUS, OHIO, US; retrieved from STN-INTERNATIONAL, accession no. 134:168324 CA XP002190403 & CN 1 258 448 A (TIANJIN AIHUA FESHENER) 5. Juli 2000 (2000-07-05)
- DATABASE WPI Section Ch, Week 199318 Derwent Publications Ltd., London, GB; Class A97, AN 1993-149118 XP002190407 & JP 05 085905 A (XYENCE KK), 6. April 1993 (1993-04-06)

## Beschreibung

Die Erfindung betrifft synergistische Desinfektionsmittelzusammensetzungen auf Basis von Aminen.

Es sind zahlreiche Desinfektions- und Konservierungsmittelzusammensetzungen auf Basis von Aminen und/oder quartären Ammoniumsalzen bekannt. Diese weisen jedoch im allgemeinen, insbesondere bei höheren Verdünnungen, eine unbefriedigende Wirksamkeit gegen Pilze wie z. B. *Aspergillus niger* auf.

Zusammensetzungen, die (neben anderen Bestandteilen) quartäre Ammoniumsalze und Ethanolamine enthalten, sind im Stand der Technik mehrfach beschrieben. CN-A-1222566 offenbart antiseptische Flüssigwaschmittel mit Dodecyldimethylbenzylammoniumchlorid und Triethanolamin.

JP-A-10 087410 und JP-A-50 132126 offenbaren Desinfektionsmittel mit Benzalkoniumchlorid bzw. Benzalkoniumhalogeniden und Triethanolamin.

JP-A-05 085905 beschreibt Holzschutzmittel mit quartären Ammoniumsalzen und Alkanolaminen.

CN-A-1258448 beschreibt Mittel zur Händedesinfektion, die quartäre Ammoniumsalze und Triethanolamin enthalten.

RO-B-87920 offenbart Antistatikmittel für synthetische Fasern, die quartäre Ammoniumsalze und Triethanolamin enthalten können.

WO-A-93/15173 beschreibt flüssige Reinigungsmittel, die quartäre Ammoniumsalze und Monoethanolamin und/oder andere β-Aminoalkanole enthalten. WO-A-98/20732 offenbart wässrige mykobakterizide Zusammensetzungen mit quartären Ammoniumsalzen, die als pH-Regulator zusätzlich Triethanolamin enthalten können. EP-A-1025967 beschreibt Holzschutzmittel, die quartäre Ammoniumsalze und Alkanolamine wie Ethanolamin enthalten.

FR-A-2602955 offenbart Reinigungs- und Desinfektionsmittel für Melkgeschirre und dergleichen, welche ein Alkyl-alkylendiamin und gegebenenfalls ein Ethanolamin enthalten. Aufgabe der vorliegenden Erfindung war daher die Bereitstellung von Desinfektionsmittelzusammensetzungen auf Basis von Aminen, welche auch bei hoher Verdünnung eine gute Wirksamkeit gegen Pilze aufweisen.

Erfindungsgemäss wird diese Aufgabe durch die Desinfektionsmittelzusammensetzung nach Patentanspruch 1 gelöst.

Es wurde überraschend gefunden, dass Amine der allgemeinen Formel 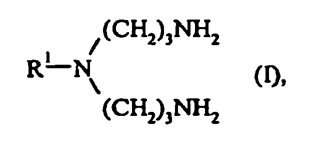 worin R¹ C₆₋₁₈-Alkyl bedeutet;
durch Zusatz von wenigstens einem Alkanolamin (II) eine gute fungizide Wirksamkeit erhalten.

Unter Alkyl sind hier und im folgenden jeweils lineare oder verzweigte Alkylgruppen der angegebenen Kohlenstoffzahlen zu verstehen, vorzugsweise jedoch lineare Alkylgruppen und besonders bevorzugt solche mit gerader Zahl von Kohlenstoffatomen. Insbesondere sind hierunter auch die von natürlichen Rohstoffen abgeleiteten Homologengemische wie beispielsweise "Kokosalkyl" zu verstehen.

Das Amin (I) ist vorzugsweise *N,N*-Bis(3-aminopropyl)dodecylamin, *N,N*-Bis(3-aminopropyl)octylamin oder ein Gemisch dieser Verbindungen.

Als Alkanolamine (II) eignen sich Monoethanolamin, Diethanolamin und 3-Amino-1-propanol. Es liegt selbstverständlich auch im Rahmen der Erfindung, Gemische der genannten Verbindungen einzusetzen. Besonders gute Ergebnisse wurden mit den Verbindungen mit primärer Aminogruppe erhalten, nämlich mit Monoethanolamin und 3-Amino-1-propanol.

Das Massenverhältnis von Amin (I) zu Alkanolamin (II) liegt im Bereich von 1:5 bis 5: 1.

Die erfindungsgemässen Desinfektionsmittelzusammensetzungen enthalten vorzugsweise Wasser als Lösungsmittel, gegebenenfalls in Kombination mit einem organischen Lösungsmittel.

Vorzugsweise enthalten die erfindungsgemässen Desinfektionsmittelzusammensetzungen noch einen oder mehrere Hilfsstoffe aus der Gruppe bestehend aus organischen Lösungsmitteln, Tensiden, Komplexbildnern, Duftstoffen und Farbstoffen.

Ein bevorzugtes Anwendungsgebiet der erfindungsgemässen Desinfektionsmittelzusammensetzungen ist die Flächen- und Instrumentendesinfektion.

Weitere bevorzugte Anwendungsgebiete sind die Wäschedesinfektion und die Händedesinfektion.

Die erfindungsgemässen Desinfektionsmittelzusammensetzungen eignen sich auch gut für den Einsatz in chemischen Toiletten wie beispielsweise an Bord von Flugzeugen und Fahrzeugen.

Ein weiteres bevorzugtes Einsatzgebiet ist die Konservierung von technischen Flüssigkeiten wie beispielsweise Wasserkreisläufe bei der Papierherstellung, Kühlwasser, Bandschmiermittel für Transportbänder oder Kühlschmierstoffe bei der Metallbearbeitung.

Eine ebenfalls bevorzugte Anwendung ist schliesslich der Einsatz als Schutz- und Konservierungsmittel für organische oder biologisch angreifbare Konstruktionsmaterialien wie beispielsweise Holz.

Die folgenden Beispiele verdeutlichen die Ausführung der Erfindung, Alle Mengenangaben sind, soweit nicht anders angegeben, in Massen-%. Als Testkeim wurde jeweils *Aspergillus niger* ATCC 16404 eingesetzt. Die Wirksamkeit wurde, soweit nichts anderes angegeben ist, nach dem in CEN 1275 spezifizierten Verfahren bestimmt.

### Beispiel 1

Es wurde eine Desinfektionsreinigerformulierung (Konzentrat) hergestellt aus:

| | |
|---|---|
| 5,0% | Didecyldimethylammoniumchlorid (50%ige Lösung) |
| 2,0% | *N,N*-Bis(3-aminopropyl)dodecylamin |
| 5,0% | Monoethanolamin |
| 5,0% | Genapol^{®} T250 (Talgfettalkoholpolyglycolether, 25 mol Ethylenoxid) |
| 0,5% | Natriummetasilicat |
| 0,5% | Natriumcarbonat |
| 2,0% | Methylglycindiessigsäure-Trinatriumsalz (Trilon^{®} M; 40%ige Lösung) Wasser ad 100% |

Die Wirksamkeit wurde mit einer Verdünnung (1 Teil Konzentrat, 99 Teile Wasser) bei 20 °C und 15 min Kontaktzeit bestimmt. Der dekadische Logarithmus der Keimzahlreduktion war 4,1.

### Vergleichsbeispiel 1

Es wurde wie in Beispiel 1 verfahren, jedoch mit dem Unterschied, dass das Monoethanolamin durch die gleiche Menge Wasser ersetzt wurde. Unter den gleichen Testbedingungen war die Formulierung praktisch unwirksam.

### Beispiel 2

Es wurde eine Desinfektionsmittelformulierung (Konzentrat) hergestellt aus:

| | |
|---|---|
| 4,9% | *N,N*-Bis(3-aminopropyl)dodecylamin |
| 4,0% | Monoethanolamin |
| 2,0% | Genapol^{®} T250 (Talgfettalkoholpolyglycolether, 25 mol Ethylenoxid) |
| 5,0% | Hostapur^{®} SAS 30 (C₁₃₋₁₇ sekundäre n-Alkansulfonsäure, Natriumsalz) |
| 2,0% | Ethylendiamintetraessigsäure-Tetranatriumsalz (40%ige Lösung) |
| 0,7% | Ethylendiamintetraessigsäure Wasser ad 100% |

Die Wirksamkeit wurde mit einer Verdünnung (1 Teil Konzentrat, 199 Teile Wasser) bei 20°C und 15 min Kontaktzeit bestimmt. Der dekadische Logarithmus der Keimzahlreduktion war 4,3.

### Beispiel 3

Es wurde eine Desinfektionsmittelformulierung (Konzentrat) hergestellt aus:

| | |
|---|---|
| 4,2% | *N,N*-Bis(3-aminopropyl)dodecylamin |
| 2,0% | Didecyl-methyl-poly(oxyethyl)ammoniumpropionat (BARDAP 26) |
| 4,0% | Monoethanolamin |
| 2,0% | Genapol^{®} T250 (Talgfettalkoholpolyglycolether, 25 mol Ethylenoxid) |
| 5,0% | Hostapur^{®} SAS 30 (C₁₃₋₁₇ sekundäre n-Alkansulfonsäure, Natriumsalz) |
| 2,0% | Ethylendiamintetraessigsäure- Tetranatriumsalz (40%ige Lösung) |
| 0,7% | Ethylendiamintetraessigsäure |
| 4,0% | Butyldiglycol Wasser ad 100% |

Die Wirksamkeit wurde mit einer Verdünnung (1 Teil Konzentrat, 199 Teile Wasser) bei 20°C und 15 min Kontaktzeit bestimmt. Der dekadische Logarithmus der Keimzahlreduktion war >4,4.
Zusätzlich wurde die Wirksamkeit noch nach dem in CEN 1650 spezifizierten Verfahren bei einer Kontaktzeit von 15 min, einer Konzentration von 1,0%, einer Wasserhärte von 30 °fH und einer organischen Belastung von 0,3% Albumin bestimmt. Der Logarithmus der Keimzahlreduktion war >4,4.

### Beispiele 4-6

Es wurden wässrige Lösungen aus 0,5% Alkanolamin (II) und 0,25% Amin (I) hergestellt und nach dem in CEN 1275 spezifizierten Verfahren gestestet. Die Ergebnisse sind in Tabelle 1 zusammengefasst.

**Tabelle 1**

| **Beispiel Nr.** | **Amin** | **Alkanolamin** | **Ig Keimreduktion** |
|---|---|---|---|
| 4 | *N,N*-Bis(3-aminopropyl)dodecylamin | Monoethanolamin | 2,9 |
| 5 | dto. | Diethanolamin | 2,7 |
| 6 | dto. | 3-Amino-1-propanol | 2,8 |

Zum Vergleich wurden alle in Tabelle 1 aufgeführten Verbindungen als Einzelsubstanzen in 0,5%iger Lösung getestet. Keine dieser Verbindungen wies eine ausgeprägte fungizide Wirkung auf (1g Keimreduktion <2).

## Patentansprüche

1. Desinfektionsmittelzusammensetzung, enthaltend
a) ein Amin der allgemeinen Formel 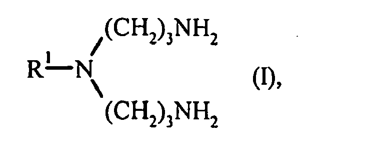 worin R¹ C₆₋₁₈-Alkyl, bedeutet; und
b) wenigstens ein Alkanolamin (II), ausgewählt aus der Gruppe bestehend aus Mono ethanolamin, Diethanolamin und 3-Amino-1-propanol
im Massenverhältnis (I):(II) von 1:5 bis 5:1.

2. Desinfektionsmittelzusammensetzung nach Anspruch 1, **dadurch gekennzeichnet, dass** das Amin ausgewählt ist aus der Gruppe bestehend aus *N,N*-Bis(3-aminopropyl)-dodecylamin und N,N-Bis(3-aminopropyl)octylamin sowie Gemischen dieser Verbindungen.

3. Desinfektionsmittelzusammensetzung nach Anspruch 1 oder 2, **dadurch gekennzeichnet, dass** sie Wasser als Lösungsmittel enthält.

4. Desinfektionsmittelzusammensetzung nach einem der Ansprüche 1 bis 3, **dadurch gekennzeichnet, dass** sie zusätzlich einen oder mehrere Hilfsstoffe aus der Gruppe bestehend aus organischen Lösungsmitteln, Tensiden, Komplexbildnern, Duftstoffen und Farbstoffen enthält.

5. Verwendung der Desinfektionsmittelzusammensetzung gemäss Ansprüchen 1 bis 4 zur Flächen- und Instrumentendesinfektion.

6. Verwendung der Desinfektionsmittelzusammensetzungen gemäss Ansprüchen 1 bis 4 zur Wäschedesinfektion.

7. Verwendung der Desinfektionsmittelzusammensetzungen gemäss Ansprüchen 1 bis 4 zur Händedesinfektion.

8. Verwendung der Desinfektionsmittelzusammensetzung gemäss Ansprüchen 1 bis 4 in chemischen Toiletten.

9. Verwendung der Desinfektionsmittelzusammensetzungen gemäss Ansprüchen 1 bis 4 als Konservierungsmittel für technische Flüssigkeiten.

10. Verwendung der Desinfektionsmittelzusammensetzung gemäss Ansprüchen 1 bis 4 als Schutz- und Konservierungsmittel für Konstruktionsmaterialien.

## Claims

1. Disinfectant composition comprising
a) an amine of the general formula 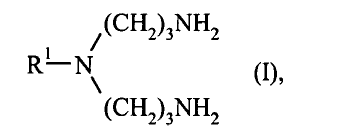 where R¹ is C₆₋₁₈-alkyl; and
at least one alkanolamine (II) selected from the group consisting of monoethanolamine, diethanolamine and 3-amino-1-propanol
in the mass ratio (I):(II) of 1:5 to 5:1.

2. Disinfectant composition according to Claim 1, **characterized in that** the amine is selected from the group consisting of *N,N*-bis(3-aminopropyl)dodecylamine, *N,N*-bis(3-aminopropyl)-octylamine, and mixtures of these compounds.

3. Disinfectant composition according to Claim 1 or 2, **characterized in that** it comprises water as solvent.

4. Disinfectant composition according to one of Claims 1 to 3, **characterized in that** it additionally comprises one or more aids selected from the group consisting of organic solvents, surfactants, complexing agents, fragrances and colorants.

5. Use of the disinfectant composition according to Claims 1 to 4 for surface disinfection and instrument disinfection.

6. Use of the disinfectant compositions according to Claims 1 to 4 for laundry disinfection.

7. Use of the disinfectant compositions according to Claims 1 to 4 for hand disinfection.

8. Use of the disinfectant composition according to Claims 1 to 4 in chemical toilets.

9. Use of the disinfectant compositions according to Claims 1 to 4 as preservatives for industrial liquids.

10. Use of the disinfectant composition according to Claims 1 to 4 as preservatives for construction materials.

## Revendications

1. Composition de désinfectant, contenant a) une amine de formule générale 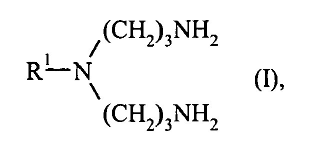 dans laquelle R¹ signifie alkyle en C₆ à C₁₈ ; au moins une alcanolamine (II) choisie dans le groupe constitué par la monoéthanolamine, la diéthanolamine et le 3-amino-1-propanol
dans un rapport massique (I):(II) de 1:5 à 5:1.

2. Composition de désinfectant selon la revendication 1, **caractérisée en ce que** l'amine est choisie dans le groupe constitué par la *N,N*-bis(3-aminopropyl)-dodécylamine et la *N,N*-bis(3-aminopropyl)octylamine ainsi que les mélanges de ces composés.

3. Composition de désinfectant selon la revendication 1 ou 2, **caractérisée en ce qu'**elle contient de l'eau comme solvant.

4. Composition de désinfectant selon l'une quelconque des revendications 1 à 3, **caractérisée en ce qu'**elle contient en outre un ou plusieurs adjuvants du groupe constitué par les solvants organiques, les agents tensioactifs, les complexants, les parfums et les colorants.

5. Utilisation de la composition de désinfectant selon les revendications 1 à 4 pour la désinfection de surfaces et d'instruments.

6. Utilisation de la composition de désinfectant selon les revendications 1 à 4 pour la désinfection du linge.

7. Utilisation de la composition de désinfectant selon les revendications 1 à 4 pour la désinfection des mains.

8. Utilisation de la composition de désinfectant selon les revendications 1 à 4 dans les toilettes chimiques.

9. Utilisation des compositions de désinfectant selon les revendications 1 à 4 comme conservateurs pour les liquides techniques.

10. Utilisation de la composition de désinfectant selon les revendications 1 à 4 comme agent de protection et conservateur pour les matériaux de construction.
